# EUROPEAN PATENT APPLICATION

(11) **EP 4 729 081 A1**
(43) Date of publication of application: **22.04.2026**
(21) Application number: 24206926.8
(22) Date of filing: 16.10.2024
(51) Int. Cl.: A61M 1/00

(54) **NEGATIVE PRESSURE WOUND THERAPY**

(71) Applicant: Mölnlycke Health Care AB, 402 52 Göteborg (SE)
(72) Inventor: BENGTSSON, Erik, 431 41 Mölndal (SE); VANNAS, Alexander, 421 51 Göteborg (SE); CARLSSON, Matthias, 431 68 Mölndal (SE); FLACH, Niclas, 441 35 Alingsås (SE); UTTERBERG, Hanna, 429 44 Särö (SE)
(74) Representative: Zacco Sweden AB

(57) **Abstract**

An apparatus and related aspects for providing reduced pressure to a tissue site are disclosed. The apparatus comprises a source of negative pressure configured to provide negative pressure via a first fluid flow path from an inlet of the source of negative pressure to a wound site, and an electronically controllable valve configured to release negative pressure from the wound dressing via a second fluid flow path. The apparatus further comprises a first pressure sensor configured to monitor a pressure level in the first fluid flow path, a second pressure sensor configured to monitor a pressure level in the second fluid flow path, and control circuitry. The control circuitry is configured to control activation and deactivation of the source of negative pressure during initial depressurization based on pressure measurements of the second pressure sensor while disregarding pressure measurements from the first pressure sensor. The control circuitry is further configured to control activation and deactivation of the source of negative pressure during pressure regulation based on pressure measurements of the second pressure sensor while disregarding pressure measurements from the first pressure sensor. The control circuitry is further configured to execute a flushing sequence by opening the electronically controllable valve so to release negative pressure from the wound site, closing the electronically controllable valve in response to reaching a first pressure threshold as measured by the first pressure sensor, activating the source of negative pressure, and deactivating the source of negative pressure in response to reaching a second pressure threshold as measured by the second pressure sensor.

## Description

### TECHNICAL FIELD

The herein disclosed embodiments generally relate to negative pressure wound therapy (NPWT). In particular, the herein disclosed embodiments relate to apparatuses for providing reduced pressure to a wound site, methods for controlling an apparatus for providing reduced pressure to a wound site, and thereto related computer program products, computer-readable storage media, and wound treatment systems.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

### SUMMARY

The herein disclosed technology seeks to mitigate, alleviate or eliminate one or more of the above-identified deficiencies and disadvantages in the prior art to address various problems relating to blockage detection in an NPWT system.

Various aspects and embodiments of the disclosed technology are defined below and in the accompanying independent and dependent claims.

An aspect of the disclosed technology comprises an apparatus for providing negative pressure to a wound site. The apparatus comprises a source of negative pressure configured to provide negative pressure via a first fluid flow path from an inlet of the source of negative pressure to a wound site, and an electronically controllable valve configured to release negative pressure from the wound dressing via a second fluid flow path. The apparatus further comprises a first pressure sensor configured to monitor a pressure level in the first fluid flow path, a second pressure sensor configured to monitor a pressure level in the second fluid flow path, and control circuitry operatively connected to the source of negative pressure, the electronically controllable valve, the first pressure sensor, and the second pressure sensor.

The control circuitry is configured to control activation and deactivation of the source of negative pressure during initial depressurization where the pressure at the wound site is brought from ambient pressure to a set negative pressure value based on pressure measurements of the second pressure sensor while disregarding pressure measurements from the first pressure sensor. The control circuitry is further configured to control activation and deactivation of the source of negative pressure during pressure regulation so to maintain pressure at a set negative pressure value or within a range of negative pressure values based on pressure measurements of the second pressure sensor while disregarding pressure measurements from the first pressure sensor. The control circuitry is further configured to execute a flushing sequence so to purge fluid from the wound site. The flushing sequence comprises opening the electronically controllable valve so to release negative pressure from the wound site, closing the electronically controllable valve in response to reaching a first pressure threshold as measured by the first pressure sensor, activating the source of negative pressure, and deactivating the source of negative pressure in response to reaching a second pressure threshold as measured by the second pressure sensor. The second pressure threshold is lower than the first pressure threshold.

Another aspect of the disclosed technology comprises a computer-implemented method for operating an apparatus for providing negative pressure to a wound site. The apparatus comprises a source of negative pressure configured to provide negative pressure via a first fluid flow path from an inlet of the source of negative pressure to a wound site, a first pressure sensor configured to monitor a pressure level in the first fluid flow path, an electronically controllable valve configured to release negative pressure from the wound site via a second fluid flow path, and a second pressure sensor configured to monitor a pressure level in the second fluid flow path. The method comprises controlling activation and deactivation of the source of negative pressure during initial depressurization where the pressure at the wound site is brought from ambient pressure to a set negative pressure value in based on pressure measurements of the second pressure sensor while disregarding pressure measurements from the first pressure sensor. The method further comprises controlling activation and deactivation of the source of negative pressure during pressure regulation so to maintain pressure at a set negative pressure value or within a range of negative pressure values based on pressure measurements of the second pressure sensor while disregarding pressure measurements from the first pressure sensor. The method further comprises executing a flushing sequence so to purge fluid from the wound site. The flushing sequence comprises opening the electronically controllable valve so to release negative pressure from the wound site, closing the electronically controllable valve in response to reaching a first pressure threshold as measured by the first pressure sensor, activating the source of negative pressure, and deactivating the source of negative pressure in response to reaching a second pressure threshold as measured by the second pressure sensor. The second pressure threshold is lower than the first pressure threshold. With this aspect of the disclosed technology, similar advantages and preferred features are present as in the other aspects.

Another aspect of the disclosed technology comprises a computer program product comprising instructions which, when the program is executed by a computing device of an apparatus for providing reduced pressure to a wound site or wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing reduced pressure to a wound site or wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a wound treatment system comprising an apparatus according to any one of the embodiments disclosed herein, a wound cover for creating a sealed space defined in part by the wound site, and a tubing assembly defining the first fluid flow path and the second fluid flow path.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that the pressure readings used as input for various therapy control functions are more representative of the pressure level at the actual wound site, thereby an improved overall therapy may be achieved.

An advantage of some embodiments is that one may obtain more accurate pressure readings for various therapy control functions without requiring a pressure sensor to be provided at the wound site, thereby a less complex and more cost-effective wound therapy system may be obtained.

An advantage of some embodiments is that an improved exudate transport may be achieved as the risk of prematurely closing the valve during the flushing sequence is reduced.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic graph of negative pressure over time as measured by a pressure sensor of an apparatus for providing negative pressure to a wound site in accordance with some embodiments.
Fig. 3 is a schematic graph of negative pressure over time as measured by a pressure sensor of an apparatus for providing negative pressure to a wound site in accordance with some embodiments.
Fig. 4 is a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The term "operatively connected" is in the context of the present disclosure to be understood as that the "operatively connected" entities or units can transmit and/or receive signals to and/or from each other.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

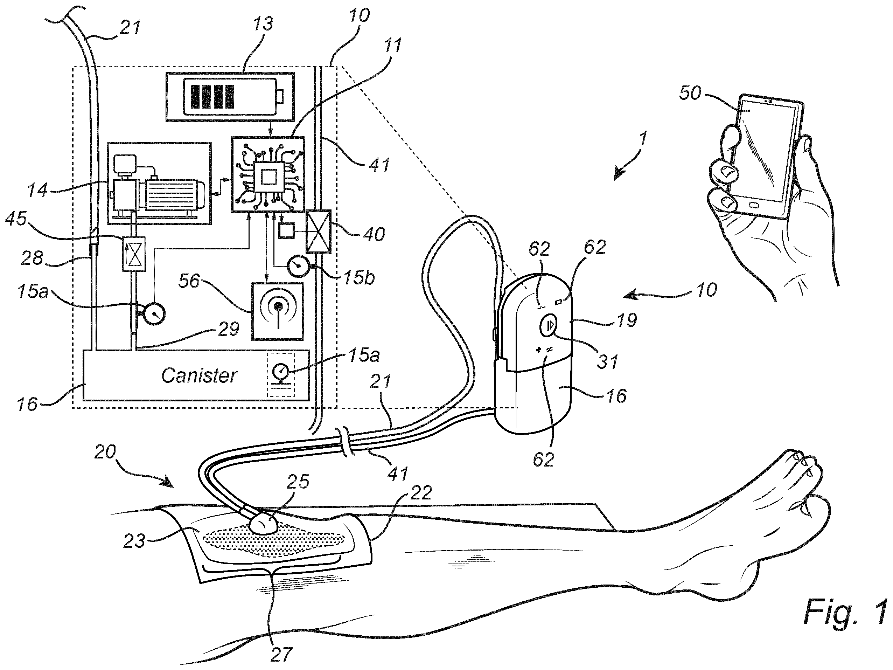

Turning now to the drawings, and to Fig. 1 in particular, there is schematically illustrated a wound treatment system 1 (sometimes simply referred to as "the system" 1) in accordance with some embodiments. The depicted wound treatment system 1 may be referred to as an "NPWT system" 1, "reduced pressure wound treatment system" 1, or "negative pressure wound treatment system" 1. In particular, the depicted wound treatment system 1 may be referred to as a dual lumen wound treatment system 1. The wound treatment system 1 comprises an apparatus 10 for providing reduced pressure to a tissue site 27 (or otherwise referred to as a wound site 27).

Further, the apparatus 10 (may also be referred to as an "NPWT device") is fluidly connected to the wound cover 22 using a tubing assembly with a first tubing 21 at least partly defining a first fluid flow path and a second tubing 41 at least partly defining a second fluid flow path. The tubing 21, 41 may be of any suitable flexible tubing fabricated from elastomeric or polymeric materials. The tubing 21, 41 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing assembly may accordingly comprise two individual tubes 21, 41.

The expression "wound cover" as used herein should be interpreted broadly as any wound site member, e.g. it can be a film sealed around a periphery of a wound site 27, where a wound filler may be used to fill the wound volume prior to the application of such wound cover 22. Wound cover 22 may also refer to a backing layer of a wound dressing 20 comprising an additional layer(s) such as for example an absorbent layer, fluid distribution layer, and/or a spacer layer.

The apparatus 10 comprises a source of negative pressure 14 ("pump device") configured to provide negative pressure (i.e. sub-atmospheric pressure) via a first fluid flow path from an inlet of the source of negative pressure 14 to the wound dressing 20, the wound site 27, the sealed space 23, or under the wound cover 22. Thus, the term "to provide negative pressure via a first fluid flow path to a wound site" may be understood as "to provide negative pressure to a wound dressing, "to provide negative pressure to a sealed space defined by a wound cover and a wound site", or "to provide negative pressure under a wound cover". The first fluid flow path ("exudate lumen") is at least partly defined by the first tubing 21.

The wound treatment system 1 further comprises a second fluid flow path that fluidly connects the wound site 27, or the sealed space 23 created by the wound cover 22, to the ambient atmosphere or a fluid reservoir (not shown) via an electronically controllable valve 40. The second fluid flow path ("air lumen") is at least partly defined by the second tubing 41. Accordingly, the fluid flow path from the inlet of the source of negative pressure 14 to the wound site 27 may be construed as a first fluid flow path or "exudate lumen", while the fluid flow path from the ambient atmosphere or fluid reservoir to the wound site 27 via the electronically controllable valve 40 may be construed as a second fluid flow path or "air lumen".

In some embodiments, the source of negative pressure 14 comprises a negative pressure pump that together with a motor is adapted for establishing a negative pressure when the source of negative pressure 14 is operating, i.e. when it is turned on, in an active state, or simply "active". The source of negative pressure 14 may comprise any type of pump and motor that are biocompatible or otherwise suitable for use in an NPWT setting and capable of maintaining or drawing adequate and therapeutic vacuum levels. Preferably, the negative pressure level to be achieved is a value within a range between about -20 mmHg and about -300 mmHg. In some embodiments, a negative pressure range between about -80 mmHg and about -140 mmHg is used. In some embodiments, a negative pressure range between about -115 mmHg and about -135 mmHg is used. In some embodiments, the negative pressure pump is a diaphragm pump, a peristaltic pump, or the like, in which a motor or actuator (e.g., piezoelectric actuator) causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in (absolute) pressure, while decreases in negative pressure typically refer to an increase in (absolute) pressure, and vice versa.

Further, in some embodiments the apparatus 10 comprises a canister 16 fluidly connected to the negative pressure source 14. In other words, the negative pressure source 14 is fluidly coupled to the canister 16 and configured to draw a negative pressure within the canister 16. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between two components such as between the canister 16 and the negative pressure source 14 or the canister 16 and the wound dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path. Furthermore, the apparatus 10 may comprise a one-way valve 45 between the canister 16 and the source of negative pressure 14 to prevent involuntary backflow of air from the source of negative pressure 14 to the wound site 27.

In some embodiments, the apparatus comprises a housing 19 enclosing the negative pressure source 14. In some embodiments, the housing 19 encloses the source of negative pressure 14, the electronically controllable valve 40, the first pressure sensor 15a, the second pressure sensor 15b, and the control circuitry 11. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

The apparatus 10 further comprises control circuitry 11 (may also be referred to as "a control unit", "a controller", or the like) operatively connected to the battery 13, the source of negative pressure 14, the electronically controllable valve 40, and the first and second pressure sensors 15a, 15b. The control circuitry 11 is configured to control operation of the source of negative pressure 14 and the electronically controllable valve 40. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor(s) 11 whereby the apparatus 10 is operative to execute any one of the method steps or functions disclosed herein. The term "electronically controllable" should in present context be understood as that one can control the unit or component that is "electronically controllable" with control signals (e.g., electric signals, optical signals, or the like), and in particular with control signals that are output from the control circuitry 11.

In some embodiments, the apparatus 10 comprises power supply circuitry 13 comprising a power source, such as e.g. a battery for powering the apparatus 10. The battery may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

The power supply circuitry 13 may further comprise suitable components to regulate the supply voltage that is provided to various components of the apparatus 10, such as to the source of negative pressure 14, the general control circuitry 11, and so forth. For example, the power supply circuitry 13 may comprise one or more voltage regulators/converters (e.g., boost regulators or buck-boost regulators). However, in some embodiments, the voltage regulators/converters are provided as a part of the control circuitry 11 depending on requirements. However, in cases where the voltage regulators/converters are provided as separate components to the control circuitry 11, they are still operatively connected to the control circuitry 11 and therefore controlled by the control circuitry 11.

Furthermore, the apparatus 10 comprises a first pressure sensor 15a configured to detect, measure, or sense a pressure level in the first fluid flow path ("exudate lumen"). Moreover, the first pressure sensor 15a arranged in fluid connection with the inlet of the source of negative pressure 14 or the canister 16. In some embodiments, the first pressure sensor 15a may be configured to monitor a pressure level proximal to the inlet of the source of negative pressure 15 at a location upstream of the inlet of the source of negative pressure 14 within the first fluid flow path (i.e., towards the wound site 27 from the source of negative pressure 14). In particular, the first pressure sensor 15a may be arranged proximal to the inlet of the source of negative pressure 14 (e.g., in connection with a flow path between the canister 16 and the source of negative pressure 14). In the depicted embodiment of Fig. 1, the apparatus 10 comprises a first pressure sensor 15a arranged within the housing 19 in a fluid flow path between the canister 16 and the source of negative pressure 14 (i.e., in fluid connection with the first fluid flow path). However, as readily understood by a person skilled in the art, the first pressure sensor 15a may be located at any other suitable location to sense or detect a pressure within the fluid flow path between the source of negative pressure 14 and the wound site 27. For example, the first pressure sensor 15a may be arranged within the canister 16 as indicated by the broken line box in Fig. 1.

The apparatus may 10 further comprise a second pressure sensor 15b arranged in fluid connection with the second fluid flow path ("air lumen"). In particular, the apparatus 10 may comprise a second pressure sensor 15b configured to detect, measure, or sense a pressure level in the second fluid flow path. In the depicted embodiment the second pressure sensor 15b is arranged within the housing 19 downstream of the electronically controllable valve 40 (i.e., towards the wound site 27 from the valve 40). In some embodiments, the second pressure sensor 15b is arranged within the housing 19, downstream of and in proximity to the electronically controllable valve 40. However, as readily understood by the skilled person in the art, the second pressure sensors 15b may be arranged at any other suitable location to detect, measure or sense a pressure level within the second fluid flow path ("air lumen"). In the following, the first pressure sensor 15a may be referred to as an "exudate lumen sensor" and the second pressure sensor 15b may be referred to as an "air lumen sensor".

During use of the apparatus 10, the wound cover 22 is arranged at a wound site 27 of the user/patient, forming the sealed space 23. A tubing assembly is provided with a first tubing 21 to fluidly connect the wound cover 22 to the inlet port 28 of the apparatus 10. The apparatus 10 is then activated, e.g. by a user pressing a start/pause button 31 of a user-interface of the apparatus 10 that activates the source of negative pressure 14. Alternatively, activation may be initiated via an external device 50 connected to the system 1. The apparatus 10 may for example comprise a suitable communication interface 56 to wirelessly connect to the external device 50. When activated, the source of negative pressure 14 will start to evacuate air through the canister 16, the inlet port 28, the tubing 21 and the sealed space 23 formed by the wound cover 22.

Accordingly, a negative pressure will be created within the sealed space 23. This initial evacuation of air following a start-up of the apparatus 10 may be referred to as "depressurization". The initial evacuation of air (i.e., "depressurization") may continue until reaching a set pressure value (e.g., -125 mmHg). In some embodiments, the control circuitry 11 is configured to control the source of negative pressure 14 so to establish and maintain a negative pressure level (as measured by the pressure sensor 15) within a negative pressure range. The negative pressure range may for example be -20 mmHg to -300 mmHg, -80 mmHg to -180 mmHg, -100 mmHg to -150 mmHg, -110 mmHg to -140 mmHg, -115 mmHg to -135 mmHg, or any other suitable range. The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (herein referred to as "exudate") that is drawn from the wound and collected in the canister 16 will depend on the type of wound that is being treated, the duration of the treatment, as well as the type of wound dressing 20 used. For example, in case an absorbent dressing is used, the exudate may be absorbed and collected both in the canister 16 and the wound dressing 20, whereas if a dressing with no absorption capacity or little absorption capacity is used, most or all of the exudate from the wound site 27 may be collected in the canister 16. A suitable filter member (not shown) is generally arranged between the canister 16 and the source of negative pressure 14, e.g., at the outlet port 29 of the canister 16 to ensure that no liquid is allowed to pass to the source of negative pressure 14 from the canister 16. In some embodiments, the filter member is arranged within the canister 14 proximal to the outlet port 29 of the canister 16.

The control circuitry 11 is further configured to control an operation of the electronically controllable valve 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing", "venting", "aerating", or "purging". Since the wound treatment system 1 depicted in Fig. 1 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1. Therefore, in order to be able to draw desired amounts of wound exudate from the wound site 27, a fluid, such as e.g. air from the ambient atmosphere, is controllably introduced at defined time intervals and/or in response to pressure measurements by opening the electronically controllable valve 40. In general, once enough fluid has been introduced (e.g. in response to a negative pressure within the system 1 reaching a lower negative pressure threshold), the control circuitry 11 is configured to close the electronically controllable valve 40 and to activate the source of negative pressure 14. However, in some embodiments, the negative pressure source 14 may be active for at least some of the duration that the electronically controllable valve 40 is open.

The ability to controllably "flush" the system 1 by injecting air via an electronically controllable valve 40 may provide the advantage of longer pressure regulation cycles (may also be referred to as NPWT cycle), which reduces the on-time for the source of negative pressure 14 and therefore reduces the energy consumption of the apparatus 10.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/min, such as 60-100 ml/min when the valve 40 is opened after negative pressure has been established under the wound cover 20. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air), or downstream of the valve 40 such as between the valve and the pressure sensor 15a.

As mentioned, the apparatus 10 may comprise a communication interface 56. The communication interface 56 may comprise suitable components (e.g. transceivers, antennas, filters, power amplifiers, etc.) with suitable communication protocols (e.g., Wi-Fi, Bluetooth, Bluetooth Low Energy, Zigbee, or the like) in order to establish a connection with an external device 50 and to transmit and receive wireless signals to and from the external device 50. The communication interface 56 is operatively connected to the control circuitry 11. The communication interface 56 may be configured to transmit and receive wireless signals to and from an external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network, cellular/mobile phone communications transceiver. In another example, the communication interface 56 may include a short-range wireless transceiver (e.g., a Bluetooth wireless transceiver, etc.) for communicating via a short-range wireless communication network. The control circuitry 11 may be configured to send one or more alarm signals or notifications indicative of a condition of the apparatus 10 (e.g., blockage alarm, leakage alarm, low battery charge alarm) to the external device 50 via the one or more antennas.

The external device may be an external handheld device 50, such as a mobile phone (e.g. a smart phone) operatively connected to the apparatus 10. Moreover, the handheld device 50 preferably comprises corresponding hardware and software capabilities to establish a wireless communication link with the apparatus 10 and to transmit and receive signals to and from the apparatus 10.

Still further, the apparatus 10 may comprise one or more Light Emitting Diodes (LEDs) 62. The one or more LEDs 62 may be considered to form a User Interface (Ul) together with the button 31 arranged on the housing 19. The LEDs 62 may for example be integrated with or arranged on the housing 19 of the apparatus 10. The LEDs 62 may be arranged under partly transparent portions of the housing to indicate various operating conditions of the apparatus 10. In more detail, the LEDs may be used to indicate a State of Charge (SoC) or available capacity of the power source 13, presence of a blockage condition, and/or a presence of a leakage condition. The aforementioned transparent portions may accordingly be formed as suitable elements/icons indicating the various operating conditions of the apparatus 10 (e.g., a battery-shaped element/icon for indicating battery capacity). Moreover, each LED may be arranged to emit light of different colours (e.g., green, yellow, red) to provide more granularity in the indications. For example, a green battery icon may indicate high battery capacity (e.g., SoC > 50%), a yellow battery icon may indicate a moderate battery capacity (e.g., 50% > SoC > 10%), while a red battery icon may indicate a low battery capacity (e.g., SoC < 10%). The apparatus 10 may comprise further LEDs than those indicated in the drawings. For example, the apparatus 10 may comprise an LED associated with the button 31 in order to indicate an active state of the apparatus 10.

It should be noted that even though the terms "exudate lumen" and "air lumen" are sometimes mentioned instead of "first fluid flow path" and "second fluid flow path". However, this should not be construed as that the first and second fluid flow paths are limited to solely consist of the tubes 21, 22 between the connector 25 and the apparatus 10 of the wound treatment system 1. For example, as depicted in Fig. 1 the first fluid flow path may comprise the first the connector 25, the first tubing 21, the canister 16, as well as any smaller tubing/manifolds/connectors between the canister 16 and the inlet of the source of negative pressure 14. Instead, the terms "exudate lumen" and "air lumen" are mainly used to increase readability, and could be read as "first fluid flow path" and "second fluid flow path".

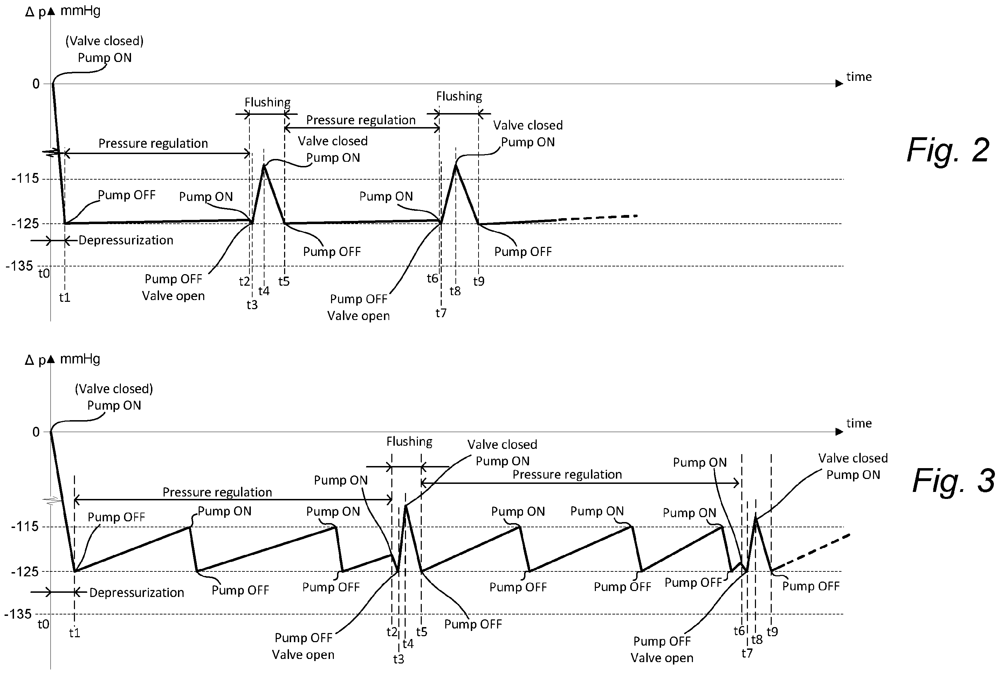

In the following, various functions of the apparatus 10, and in particular of the control circuitry 11, will be described in reference to Figs. 2 and 3, which are schematic graphs of negative pressure over time for a wound treatment system 1 in accordance with some embodiments. In more detail, Fig. 2 illustrates a "low leakage scenario" while Fig. 3 illustrates an "elevated leakage scenario" for a corresponding system 1. The "elevated leakage scenario" may be understood as that the wound treatment system 1 has some unwanted leakage ("parasitic leakage"). The unwanted leakage may for example be due to improperly applied wound cover 22 and/or leaky connections between the tubing and other components.

Moving on, when the apparatus 10 is activated, the control circuitry 11 is configured to control activation and deactivation of the source of negative pressure 14 for an "initial depressurization" where the pressure at the wound site is brought from ambient pressure to a set negative pressure value or "target pressure value". In other words, the apparatus 10 is operated in a "depressurization mode" or "depressurization state" (t0 to t1 in Fig. 2 and Fig. 3). In the embodiments depicted in Figs. 2 and 3, the set negative pressure value is -125 mmHg.

Further, once a suitable negative pressure level has been established at the wound site 27, the control circuitry 11 may be configured to regulate the negative pressure provided to the wound site over a plurality of pressure regulation cycles. In general, a pressure regulation cycle may be understood as a time period comprising at least a "pressure regulation period" and a "flushing period" (see e.g., Figs. 2-3). The "pressure regulation period" may be referred to as "inter-flushing period" and defines a time between two consecutive flushing periods (e.g., the time period from t5 to t6 in Figs. 2 and 3). The time between the end of the initial depressurization and the first flushing period (e.g., the time period from t1 to t2 in Figs. 2 and 3) may also be referred to as a pressure regulation period. During the "pressure regulation period" the control circuitry 11 may be configured to operate the negative pressure source 14 so to maintain a level of negative pressure to be within a suitable negative pressure range as exemplified above, i.e., the apparatus 10 is operated in a "pressure regulation mode" or "pressure regulation state". In other words, during the "pressure regulation period" the electronically controllable valve 40 is closed and the source of negative pressure 14 is operated to maintain a desired level of negative pressure at the wound site 27. The desired level of negative pressure may for example comprise a negative pressure range. In the embodiments depicted in Figs. 2 and 3, the control circuitry 11 is configured to control activation and deactivation of the source of negative pressure 14 during pressure regulation so to maintain pressure within the range -115 mmHg and -125 mmHg. However, the pressure range may be any pressure range depending on the specific circumstances of the patient and/or the wound. For example, in some embodiment, the control circuitry 11 is configured to control activation and deactivation of the source of negative pressure 14 during pressure regulation so to maintain pressure within the range from -115 mmHg to -135 mmHg. The range may be selected or dynamically controlled so to maintain some desired average pressure during the pressure regulation period.

During the "flushing period" the control circuitry 11 is configured to open the electronically controllable valve 40 so to introduce fluid (e.g., air from the ambient atmosphere) to the wound site 27, close the valve 40, and activate the source of negative pressure 14 to draw wound exudate from the wound site and re-establish the negative pressure level, i.e., the apparatus 10 is operated in a "flushing mode" or "flushing state". Accordingly, the flushing period causes a temporary decrease in negative pressure at the wound site 27 (i.e., a temporary increase in absolute/gauge pressure).

The duration of an inter-flushing period and a flushing period, and accordingly the duration of a pressure regulation cycle may be fixed, or in other words, time-controlled. For example, the apparatus 10 may be configured to execute a flushing sequence every 30 minutes or every hour for the entire therapy duration.

When using a dual lumen wound therapy system 1 having at least two pressure sensors, one may generally rely one the pressure readings from the air lumen sensor 15b as a "primary sensor" as it is generally considered that the pressure in the air lumen more accurately represents the pressure at the wound site 27. However, the present inventors realized that, while this may be true for most functions of the apparatus 10, it is not always suitable to use as input for certain control functions due to its proximity to the electronically controllable valve 40.

In more detail, when the valve 40 is opened, there will temporarily be a quick increase in pressure locally near the air lumen sensor 15b. This temporary and quick increase in pressure does not correspond to the actual pressure level at the wound site 27 due to the differences in the volume under the wound cover 22 and the volume of the air lumen where the air lumen sensor 15b is measuring, as well as due to the inertia due to air resistance. This may therefore result in a premature closing of the electronically controllable valve 40, and consequently in a negative impact on the therapy. For example, during the flushing sequence, if the electronically controllable valve 40 is closed prematurely, not enough air may be injected into the fluid circuit of the system 1, which impairs the moving of exudate from the wound site 27 to the canister 16. Similar reasoning may be applied for when the source of negative pressure 14 is activated with respect to the pressure readings of the exudate lumen sensor 15a, where the pressure level near the inlet of the source of negative pressure 14 is likely to be lower than the pressure level at the wound site 27 for a relatively brief period of time after the source of negative pressure 14 is activated.

To this end, it is herein proposed to introduce a functionality that shifts which pressure sensor 15a, 15b is used to control certain stages of the therapy. In other words, the pressure data input to the various control algorithms is alternated between the two pressure sensors 15a, 15b depending on the stage or sub-step of the various control algorithms that is being executed. In more detail, the output from the air lumen pressure sensor 15b is used to control the depressurization stage, the pressure regulation stage, and certain parts of the flushing stage, while the exudate lumen sensor 15a is used to control a timing of the valve closing during the flushing stage. Stated differently, the algorithms that control the depressurization, pressure regulation, and flushing rely on pressure readings from the air lumen sensor 15a up until the electronically controllable valve 40 is opened during the execution of the flushing sequence. Once the valve 40 has been opened, a temporary shift is made so that the control algorithm relies upon the pressure readings from the exudate lumen sensor 15a until the valve 40 is closed. In other words, the control algorithm relies upon pressure measurements from the exudate lumen sensor 15a in order to control the timing of the closing of the valve 40. Once the valve 40 is closed, the control algorithm relies upon the pressure readings from the air lumen sensor 15b again.

Thereby, the pressure values that the control algorithm relies upon more accurately represent the actual pressure level at the wound site 27 during all stages of the therapy, and an overall improvement in the applied therapy may be achievable. It should be note that the term "pressure at the wound site" as used herein does not require that the pressure level is measured directly at the wound site, for example by having a pressure sensor provided within the wound dressing 20. Instead, the "pressure at the wound site" is represented by the pressure values measured by the first pressure sensor 15a or the second pressure sensor 15b, as it is an isolated and closed-circuit system. It was however realized by the present inventors, that it would be advantageous to employ a functionality that allows for switching between the two pressure sensors 15a, 15b during different steps or stages of the control algorithms, as the two pressure sensors 15a, 15b may, due to their placements in the system 1, be exposed to temporary effects that negatively impact how well their pressure readings correspond to the pressure level at the wound site 27. An advantage of having the pressure sensors 15a, 15b arranged within the housing 19 of the apparatus 10 rather than the wound dressing 20 is that it alleviates the need for complex wiring schemes to electronically connect the wound dressing 20 to the apparatus 10 or having complex and costly sensors capable of wireless communication.

Accordingly, the control circuitry 11 is configured to control activation and deactivation of the source of negative pressure 14 during initial depressurization where the pressure at the wound site 27 is brought from ambient pressure to a set negative pressure value based on pressure measurements of the second pressure sensor 15b while disregarding pressure measurements from the first pressure sensor 15a. In other words, during initial depressurization (time period between t0 and t1 in Figs. 2 and 3), the pressure measurement input to the control algorithm that controls the activation and deactivation of the source of negative pressure 14, is the output from the second pressure sensor 15b (air lumen sensor) and not the output from the first pressure sensor 15a (exudate lumen sensor).

The term "while disregarding pressure measurements from sensor X" should in the present context be construed broadly, and does not necessarily imply that the pressure measurements from sensor X are treated in one specific way by the control circuitry 11. The pressure measurements from sensor X may be ignored, down-weighted, or never provided as an input to the control algorithm depending on specific hardware/software realizations. For example, sensor X may continuingly output pressure readings to the control circuitry 11, which may be configured to either not use them as input by ignoring them or to receive the pressure values and apply a zero-weight to the same. Similarly, the control circuitry may be configured to temporarily turn off sensor X if it intends to "disregard" its pressure measurements. Thus, the term "while disregarding pressure measurements from sensor X" for a certain function, may be understood as "not using pressure measurements from sensor X" for a certain function.

Further, the control circuitry 11 is configured to control activation and deactivation of the source of negative pressure 14 during pressure regulation so to maintain pressure at a set negative pressure value or within a range of negative pressure values based on pressure measurements of the second pressure sensor 15b while disregarding pressure measurements from the first pressure sensor 15a. In other words, during pressure regulation (time period between t1 and t2 or t5 and t6 in Figs. 2 and 3), the pressure measurements/input to the control algorithm that controls the activation and deactivation of the source of negative pressure 14, is the output from the second pressure sensor 15b (air lumen sensor) and not the output from the first pressure sensor 15a (exudate lumen sensor).

In some embodiments, the control circuitry 11 is configured to control activation and deactivation of the source of negative pressure 14 during pressure regulation so to maintain pressure within a range of negative pressure values (e.g., -115 mmHg and -125 mmHg as depicted in Figs. 2 and 3). In more detail, the control circuitry 11 may be configured to activate the source of negative pressure 14 in response to reaching an upper pressure threshold as measured by the second pressure sensor 15b, and deactivate the source of negative pressure 14 in response to reaching a lower pressure threshold as measured by the second pressure sensor 15b. Here, the upper pressure threshold and the lower pressure threshold define end-points of the range of negative pressure values.

Still further, the control circuitry 11 is configured to execute a flushing sequence so to purge fluid from the wound site 27. The flushing sequence comprises opening the electronically controllable valve 40 so to release negative pressure from the wound site 27, closing the electronically controllable valve 40 in response to reaching a first pressure threshold as measured by the first pressure sensor 15a, activating the source of negative pressure 14, and deactivating the source of negative pressure 14 in response to reaching a second pressure threshold as measured by the second pressure sensor 15b. Here, the second pressure threshold is lower than the first pressure threshold. The first pressure threshold may for example be -115 mmHg or -110 mmHg and the second pressure threshold may for example be -125 mmHg or -130 mmHg. An example of a resulting pressure level at the wound site 27 over time during a flushing sequence is schematically indicted between time t2 and t5 and between time t6 and t9 in Figs. 2 and 3.

In some embodiments, the flushing sequence comprises activating the source of negative pressure 14 prior to opening the electronically controllable valve 40 (see e.g., t2 and t3 in Figs. 2 and 3). In more detail, the control circuitry 11 may be configured to initiate the flushing sequence by activating the source of negative pressure and deactivating the source of negative pressure 14 (see e.g., t3 and t7 in Figs. 2 and 3) in response to reaching the second pressure threshold as measured by the second pressure sensor (air lumen sensor) 15b. Subsequently, the valve 40 may be opened and closed in response to reaching the first pressure threshold as measured by the first pressure sensor (exudate lumen sensor) 15a as mentioned above (see e.g., t4 and t8 in Figs. 2 and 3). Subsequently, the source of negative pressure 14 is activated again, and then deactivated in response to reaching the second pressure threshold as measured by the air lumen sensor 15b (see e.g., t5 and t9 in Figs. 2 and 3). The initial activation of the source of negative pressure 14 at the start of the flushing sequence may provide the advantageous effect of increasing the amount of fluid being introduced into the system 1 during flushing and thereby increase the efficacy in the purging of fluids from the wound site 27. For example, in an elevated leakage scenario (as depicted in Fig. 3), the flushing sequence may start when the pressure level is close to the first pressure threshold (the upper pressure threshold), and therefore the valve may be opened and immediately closed, leading to a potentially insufficient amount of fluid being introduced into the system 1 for an effective flushing.

Naturally, as the skilled reader realizes, the curves in Figs. 2 and 3 are schematic drawings, and that the scale and timing of certain actions in the plot may be skewed in order to improve readability. For example, the timing of the valve opening and activation of the pump at time t3 or t7 does not necessarily imply that the two actions are performed at exactly the same moment in time, but that they may occur at "substantially" the same time. In practice, the pump may be turned off some milliseconds before the valve is opened.

In some embodiments, the upper pressure threshold (used as an end-point during pressure regulation) is the same value as the first pressure threshold (defining the timing of the valve closing during the flushing sequence) and the lower pressure threshold (used as the other end-point during pressure regulation) is the same value as the second pressure threshold (defining timing the deactivation of the pump during the flushing sequence). In other words, the control circuitry 11 aims to maintain the pressure level at the wound site between the same two end-points both during pressure regulation and during flushing.

However, in some embodiments, the first pressure threshold (defining the timing of the valve closing during the flushing sequence) is higher than the upper pressure threshold (used as an end-point during pressure regulation) and the lower pressure threshold (used as the other end-point during pressure regulation) is the same value as the second pressure threshold (defining timing the deactivation of the pump during the flushing sequence). In other words, the control circuitry 11 allows the negative pressure level in the system to drop more during flushing than during pressure regulation. The first pressure threshold may for example be 5 mmHg, 10 mmHg or 15 mmHg higher than the upper pressure threshold. So, if the upper pressure threshold is -115 mmHg, the first pressure threshold may be -110 mmHg, -105 mmHg, or -100 mmHg.

Moreover, in some embodiments, the upper pressure threshold (used as an end-point during pressure regulation) is the same value as the first pressure threshold (defining the timing of the valve closing during the flushing sequence) and the lower pressure threshold (used as the other end-point during pressure regulation) is higher than the second pressure threshold (defining timing the deactivation of the pump during the flushing sequence). In other words, the control circuitry 11 allows the negative pressure level in the system during flushing to exceed the "maximum" negative pressure threshold using during pressure regulation. The second pressure threshold may for example be 5 mmHg, 10 mmHg or 15 mmHg lower than the lower pressure threshold. So, if the lower pressure threshold is -125 mmHg, the second pressure threshold may be -130 mmHg, -135 mmHg, or -140 mmHg.

By allowing the pressure threshold used during flushing to exceed either one or both of the upper and lower pressure thresholds used during regulation may allow for a larger exudate transport during flushing as more air is temporarily introduced to the system 1 and/or drawn out of the system 1.

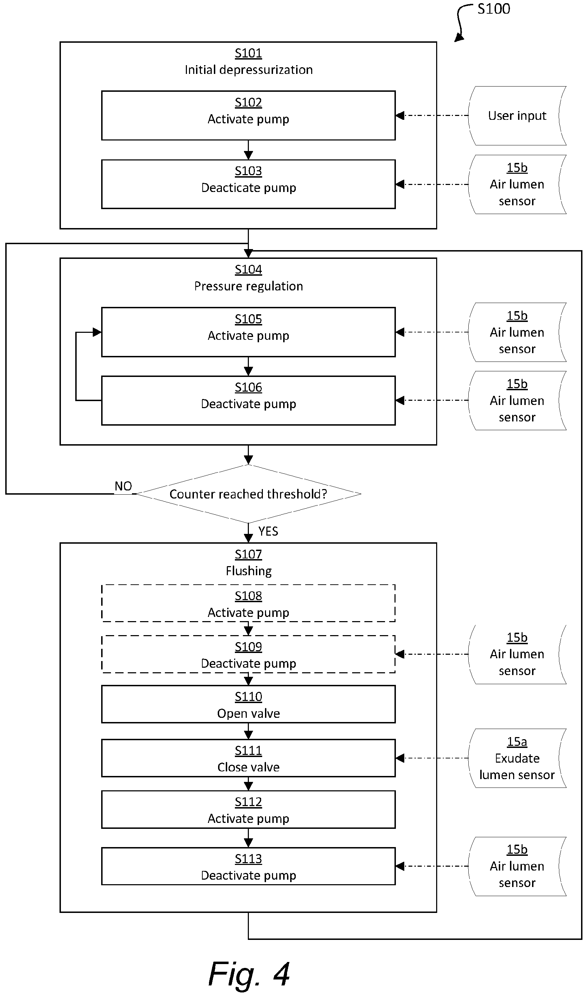

Turning now to Fig. 4, which illustrates a schematic flowchart representation of a method S100 for operating an apparatus 10 for providing negative pressure to a wound site 27 in accordance with some embodiments. The apparatus 10 may be an apparatus 10 according to any one of the embodiments disclosed herein. The method S100 is preferably a computer-implemented method S100, performed by a processing system of the apparatus 10. The processing system may for example comprise one or more processors 11 and one or more memories coupled to the one or more processors 11, wherein the one or more memories store one or more programs that perform the steps, services and functions of the method S100 disclosed herein when executed by the one or more processors.

The method S100 is illustrated as three stages or states of a control algorithm of the apparatus 10 that controls the source of negative pressure 14 and the electronically controllable valve 40. In more detail, the method S100 comprises an initial depressurization stage S101, a pressure regulation stage S104 (may also be referred to as therapy regulation stage), and a flushing stage S107. The primary input used for initiating or executing a specific sub-step of each stage is also indicated by the partially curved boxes feeding into the sub-steps in Fig. 4

Accordingly, the method S100 comprises controlling S101 activation and deactivation of the source of negative pressure during initial depressurization where the pressure at the wound site is brought from ambient pressure to a set negative pressure value in based on pressure measurements of the second pressure sensor while disregarding pressure measurements from the first pressure sensor. In more detail, the controlling S101 during the initial depressurization may comprise activating S102 the source of negative pressure in response to receiving a user-input, and deactivating S103 the source of negative pressure in response to reaching a target pressure value ("set negative pressure value") as measured by the second pressure sensor.

Further, the method S100 comprises controlling S104 activation and deactivation of the source of negative pressure during pressure regulation so to maintain a pressure level at a set negative pressure value or within a range of negative pressure values based on pressure measurements of the second pressure sensor while disregarding pressure measurements from the first pressure sensor. Accordingly, the controlling S104 during the pressure regulation stage/period may comprise activating S105 the source of negative pressure in response to reaching an upper pressure threshold as measured by the second pressure sensor, and deactivating S106 the source of negative pressure in response to reaching a lower pressure threshold as measured by the second pressure sensor. Here, the upper pressure threshold and the lower pressure threshold may define end-points of the range of negative pressure values. Moreover, in cases where the controlling S104 during the pressure regulation stage/period is done so to maintain a pressure level at a set negative pressure value the activation S105 and deactivation S106 of may be done based on historical pressure values as measured by the second pressure sensor (during the last minute, hour, day, or since first activation) so that an average pressure level as measured by the second pressure sensor (during the last minute, hour, day, or since first activation) is at the set negative pressure value.

Further, the method S100 comprises executing S107 a flushing sequence so to purge fluid from the wound site. The flushing sequence comprises opening S110 the electronically controllable valve so to release negative pressure from the wound site. The trigger for opening S110 the valve and effectively starting the flushing sequence may for example be time-controlled. For example, the flushing sequence may be triggered at regular intervals (e.g., every 30 minutes or every 60 minutes). The trigger signal may for example be provided by a suitably configured counter. Moreover, in some embodiments, the flushing sequence is initiated by an activation S108 and deactivation S109 of the source of negative pressure prior to opening the valve. Thus, in some embodiments, the flushing sequence comprises activating S108 the source of negative pressure and deactivating S109 the source of negative pressure in response to reaching a lower pressure threshold ("second pressure threshold") as measured by the second pressure sensor (air lumen sensor).

Further, following the opening S110 of the valve the flushing sequence comprises closing Sill the electronically controllable valve in response to reaching a first pressure threshold as measured by the first pressure sensor and activating S112 the source of negative pressure. The activation S112 of the source of negative pressure and the closing Sill of the valve may be executed with partial overlap. However, in some embodiments the activation S112 of the source of negative pressure and the closing Sill of the valve is performed sequentially. In other words, the source of negative pressure may be activated S112 prior to the closing Sill of the valve, or the valve may be closed S111 prior to the activation S112 of the source of negative pressure.

Moreover, the activation S112 of the source of negative pressure may be time controlled. For example, the source of negative pressure may be activated a certain time after the valve was opened S110. Thus, the trigger signal for activating S112 the source of negative pressure may be provided by a suitably configured counter. Alternatively, the source of negative pressure may be activated S112 in response to reaching some set pressure value as measured by the first pressure sensor. The trigger for closing Sill the valve is as discussed in the foregoing based on pressure readings output by the first pressure sensor.

Further, the flushing sequence comprises deactivating S113 the source of negative pressure in response to reaching a second pressure threshold as measured by the second pressure sensor. Here, the second pressure threshold is lower than the first pressure threshold. Accordingly, the flushing sequence control algorithm now switches from relying on the output from the first pressure sensor to relying on the output form the second pressure sensor again.

Executable instructions for performing these functions are, optionally, included in a non-transitory computer-readable storage medium or other computer program product configured for execution by one or more processors of an apparatus for providing reduced pressure to a wound dressing.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a reduced-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing negative pressure to a wound site (27), the apparatus comprising:
a source of negative pressure (14) configured to provide negative pressure via a first fluid flow path from an inlet of the source of negative pressure to a wound site;
an electronically controllable valve (40) configured to release negative pressure from the wound dressing via a second fluid flow path;
a first pressure sensor (15a) configured to monitor a pressure level in the first fluid flow path;
a second pressure sensor (15b) configured to monitor a pressure level in the second fluid flow path;
control circuitry (11) operatively connected to the source of negative pressure, the electronically controllable valve, the first pressure sensor, and the second pressure sensor, wherein the control circuitry is configured to:
control activation and deactivation of the source of negative pressure (14) during initial depressurization where the pressure at the wound site (27) is brought from ambient pressure to a set negative pressure value based on pressure measurements of the second pressure sensor (15b) while disregarding pressure measurements from the first pressure sensor (15a);
control activation and deactivation of the source of negative pressure (14) during pressure regulation so to maintain pressure at a set negative pressure value or within a range of negative pressure values based on pressure measurements of the second pressure sensor (15b) while disregarding pressure measurements from the first pressure sensor (15a);
execute a flushing sequence so to purge fluid from the wound site (27), the flushing sequence comprising:
opening the electronically controllable valve (40) so to release negative pressure from the wound site;
closing the electronically controllable valve (40) in response to reaching a first pressure threshold as measured by the first pressure sensor (15a);
activating the source of negative pressure (14);
deactivating the source of negative pressure (14) in response to reaching a second pressure threshold as measured by the second pressure sensor (15b), wherein the second pressure threshold is lower than the first pressure threshold.

2. The apparatus (10) according to claim 1, wherein the first pressure sensor (15a) is configured to monitor a pressure level at a location upstream of the inlet of the source of negative pressure (14) within the first fluid flow path.

3. The apparatus (10) according to claim 1 or 2, wherein the second pressure sensor (15b) is configured to monitor a pressure level at a location downstream of the electronically controllable valve (40) within the second fluid flow path.

4. The apparatus (10) according to any one of claims 1-3, further comprising a housing (19) enclosing the source of negative pressure (14), the electronically controllable valve (40), the first pressure sensor (15a), the second pressure sensor (15b), and the control circuitry (11).

5. The apparatus (10) according to any one of claims 1-4, further comprising a canister (16) defining a portion of the first fluid flow path, wherein the flushing sequence is executed so to purge fluid from the wound site (27) to the canister (16).

6. The apparatus (10) according to any one of claims 1-5, wherein the control circuitry (11) is configured to:
control activation and deactivation of the source of negative pressure (14) during pressure regulation so to maintain pressure within the range of negative pressure values by:
activating the source of negative pressure (14) in response to reaching an upper pressure threshold as measured by the second pressure sensor (15b);
deactivating the source of negative pressure (14) in response to reaching a lower pressure threshold as measured by the second pressure sensor (15b);
wherein the upper pressure threshold and the lower pressure threshold define end-points of the range of negative pressure values.

7. The apparatus (10) according to claim 6, wherein the upper pressure threshold is the same value as the first pressure threshold and the lower pressure threshold is the same value as the second pressure threshold.

8. The apparatus (10) according to claim 6, wherein the first pressure threshold is higher than the upper pressure threshold and the lower pressure threshold is the same value as the second pressure threshold.

9. A computer-implemented method (S100) for operating an apparatus for providing negative pressure to a wound site, wherein the apparatus comprises a source of negative pressure configured to provide negative pressure via a first fluid flow path from an inlet of the source of negative pressure to a wound site, a first pressure sensor configured to monitor a pressure level in the first fluid flow path, an electronically controllable valve configured to release negative pressure from the wound site via a second fluid flow path, and a second pressure sensor configured to monitor a pressure level in the second fluid flow path, the method comprising:
controlling (S101) activation and deactivation of the source of negative pressure during initial depressurization where the pressure at the wound site is brought from ambient pressure to a set negative pressure value in based on pressure measurements of the second pressure sensor while disregarding pressure measurements from the first pressure sensor;
controlling (S104) activation and deactivation of the source of negative pressure during pressure regulation so to maintain pressure at a set negative pressure value or within a range of negative pressure values based on pressure measurements of the second pressure sensor while disregarding pressure measurements from the first pressure sensor;
executing (S107) a flushing sequence so to purge fluid from the wound site, the flushing sequence comprising:
opening (S110) the electronically controllable valve so to release negative pressure from the wound site;
closing (S111) the electronically controllable valve in response to reaching a first pressure threshold as measured by the first pressure sensor;
activating (S112) the source of negative pressure;
deactivating (S113) the source of negative pressure in response to reaching a second pressure threshold as measured by the second pressure sensor, wherein the second pressure threshold is lower than the first pressure threshold.

10. The method (S100) according to claim 9, wherein controlling (S104) activation and deactivation of the source of negative pressure during pressure regulation so to maintain pressure within the range of negative pressure values comprises:
activating (S105) the source of negative pressure in response to reaching an upper pressure threshold as measured by the second pressure sensor;
deactivating (S106) the source of negative pressure in response to reaching a lower pressure threshold as measured by the second pressure sensor;
wherein the upper pressure threshold and the lower pressure threshold define end-points of the range of negative pressure values.

11. The method (S100) according to claim 10, wherein the upper pressure threshold is the same values as the first pressure threshold and the lower pressure threshold is the same value as the second pressure threshold.

12. The method (S100) according to claim 10, wherein the first pressure threshold is higher than the upper pressure threshold and the lower pressure threshold is the same value as the second pressure threshold.

13. A computer program product comprising instructions which, when the program is executed by a computing device of an apparatus for providing negative pressure to a wound site, causes the apparatus to carry out the method (S100) according to any one of claims 9-12.

14. A wound treatment system (1) comprising:
an apparatus (10) according to any one of claims 1-8;
a wound cover (22) for creating a sealed space defined in part by the wound site; and
a tubing assembly defining the first fluid flow path and the second fluid flow path.
